# EUROPEAN PATENT APPLICATION

(11) **EP 4 201 403 A1**
(43) Date of publication of application: **28.06.2023**
(21) Application number: 21383169.6
(22) Date of filing: 21.12.2021
(51) Int. Cl.: A61K 31/192, A61K 31/198, A61K 31/4025, A61K 31/407, A61K 31/439, A61K 31/445, A61K 31/517, A61K 31/53, A61P 25/00

(54) **COMPOUNDS TIRAPAZAMINE AND QUAZINONE FOR USE IN THE TREATMENT OF GM2 GANGLIOSIDOSES**

(71) Applicant: Som Innovation Biotech, S.L., 08028 Barcelona (ES)
(72) Inventor: JORBA JUEZ, Gerard, E-08028 Barcelona (ES); HUERTAS GAMBÍN, Oscar, E-08028 Barcelona (ES); INSA BORONAT, Raúl, E-08028 Barcelona (ES); REIG BOLAÑO, Núria, E-08028 Barcelona (ES); LLOYD-EVANS, Emyr, Cardiff, CF10 3AX (GB); EVANS, Charles, Cardiff, CF10 3AX (GB)
(74) Representative: ABG Intellectual Property Law, S.L.

(57) **Abstract**

The present invention relates to a compound selected from the group consisting of Tirapazamine and Quazinone, or a pharmaceutically acceptable salt thereof, and to combinations thereof, for use in the treatment or prevention of a GM2 gangliosidosis.

## Description

### FIELD OF THE INVENTION

The present invention relates to compounds for use in the treatment and/or prevention of GM2 gangliosidoses, and in particular of Tay-Sachs Disease and Sandhoff disease.

### BAKGROUND OF THE INVENTION

The GM2 gangliosidoses are a group of related disorders that result from a deficiency of the enzyme beta-hexosaminidase A (Hex A). This enzyme catalyzes the biodegradation of glycosphingolipid ganglioside GM2 (Sandhoff and Harzer, 2013). Genetic defect in the genes encoding the α and β subunits of Hex A, or the GM2 activator protein, can result in accumulation of GM2 in neural tissue leading to the three forms of GM2 gangliosidoses: Tay-Sachs (TS) disease, associated with a defect in the α-subunit, Sandhoff disease, associated with a defect in the β-subunit, and the GM2 activator protein deficiency (GM2 gangliosidosis AB variant), associated with a defect on GM2 activator protein, respectively (Bateman et al., 2011). The excessive neuronal accumulation of GM2 is accompanied by progressive neurological deterioration affecting motor, cerebral and spinocerebellar functions.

Mutations that cause a complete enzyme deficiency are extremely severe, and children affected by this form of disease usually die by age 3 or 4 years. Mutations affecting the folding or dimerization, but which are still compatible with formation of some functional enzyme and resulting in residual Hex A activity (1-5 % of normal), are less severe and result in a "late-onset" clinical phenotype (Leinekugel et al., 1992; Mahuran, 1999). It has been estimated that a residual Hex A activity of 5-10% of normal is sufficient to prevent GM2 accumulation (Conzelmann and Sandhoff, 1983). Therefore, increasing β-Hex activity provides a promising approach for treating GM2-gangliosidosis.

It has been reported (Toro et al., 2021) that the prevalence of Tay-Sachs Disease is between 1 in 200,000-300,000 and that the prevalence of Sandhoff disease is about 1 in 1,000,000. There are no authorized therapies for the treatment of the GM2 Gangliosidoses (Tay-Sachs and Sandhoff disease (Patterson, Marc C. (2013-01-01), Dulac, Olivier; Lassonde, Maryse; Sarnat, Harvey B. (eds.), "Chapter 174 - Gangliosidoses", Handbook of Clinical Neurology, Pediatric Neurology Part III, Elsevier, 113, pp. 1707-1708, retrieved 2019-08-01). The current standard of care for GM2 Gangliosidosis disease is limited to supportive care and aimed at managing symptoms and providing adequate nutrition and hydration.

It is the aim of the present invention to identify compounds capable of increasing Hex A activity to thus enrich the arsenal of therapies against GM2 gangliosidoses.

It has been shown that accumulation of GM2 gangliosides has a role in the pathology of other neurodegenerative diseases and that increasing the activity of beta-hexosaminidase may be beneficial for the treatment of Alzheimer's disease (Knight et al Molecular Psychiatry (2015) 20, 109-117; doi:10.1038/mp.2014.135) and Parkinson's disease (Brekk et al Acta Neuropathologica Communications (2020) 8:127 https://doi.org/10.1186/s40478-020-01004-6). Therefore, the compounds identified in the present invention may also be used for the treatment of other diseases that may benefit of an increase of beta-hexosaminidase activity, such as Alzheimer's disease and Parkinson's disease.

### SUMMARY OF THE INVENTION

The inventors have surprisingly found a series of compounds capable of increasing Hex A activity, and which can therefore be useful to prevent and treat GM2 gangliosidoses.

Thus, in a first aspect, the present invention relates to a compound selected from the group consisting of Tirapazamine and Quazinone, or a pharmaceutically acceptable salt thereof, for use in the treatment and/or prevention of a GM2 gangliosidosis.

In a second aspect, the invention relates to the use of a compound selected from the group consisting of Tirapazamine and Quazinone or a pharmaceutically acceptable salt thereof, in the manufacture of a medicament for the treatment and/or prevention of a GM2 gangliosidosis.

In a third aspect, the invention also relates to a method of treating and/or preventing a GM2 gangliosidosis in a subject, comprising administering to said subject a therapeutically effective amount of a compound selected from the group consisting of Tirapazamine and Quazinone, or a pharmaceutically acceptable salt thereof.

In a fourth aspect, the present invention relates to a combination comprising two or more agents selected from the group consisting of Tirapazamine, Quazinone, N-acetyl-DL-leucine, N-acetyl-L-leucine, RT-001, venglustat, miglustat, eliglustat, ACT-519276, ruboxistaurin, gemfibrozil, cinnamic acid, OT-1009, TGTX-101, TSHA-119, AAV2/1-HEXA/HEXB gene therapy, cobnabexagene anvuparvovec, DUOC-01, GT-0005X, AX-451, or a pharmaceutically acceptable salt thereof, wherein at least one of said agents is selected from the group consisting of Tirapazamine and Quazinone, or a pharmaceutically acceptable salt thereof.

In a fifth aspect, the invention relates to a combination according to the fourth aspect of the invention, for use in the treatment and/or prevention of a GM2 gangliosidosis.

In a sixth aspect, the invention relates to the use of a combination according to the fourth aspect of the invention in the manufacture of a medicament for the treatment and/or prevention of a GM2 gangliosidosis.

In a seventh aspect, the invention relates to a method of treating and/or preventing a GM2 gangliosidosis in a subject, comprising administering to said subject a therapeutically effective amount of a combination according to the fourth aspect of the invention.

### DESCRIPTION OF THE FIGURES

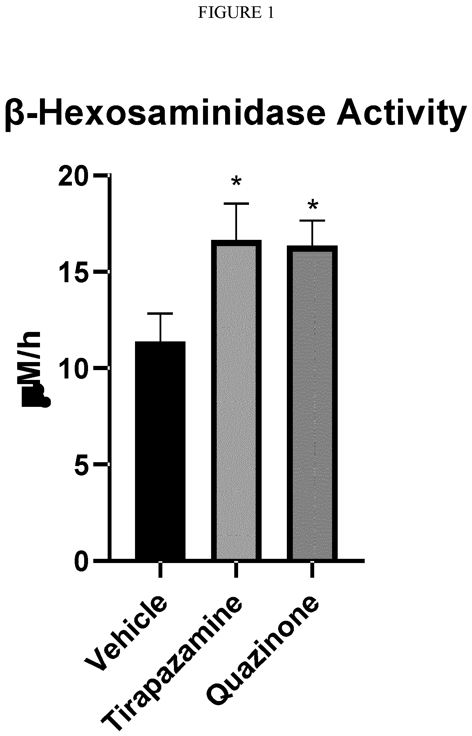

**Fig. 1** **shows the effect of vehicle (DMSO), tirapazamine and quinazone on the b-Hexosaminidase activity in Tay-Sachs disease GM0502 human fibroblasts.** b-Hex activity in TS GM0502 cells was increased following 10-days treatment with Tirapazamine or Quazinone at 3µg/ml compared to DMSO treated cells (vehicle). ^{∗}p<0.05. All bars represent the means of 3 independent samples. Error bars represent the standard deviation.

### DETAILED DESCRIPTION OF THE INVENTION

As outlined above, the present invention pertains to a series of two compounds which have been found to increase Hex A activity. These compounds are presented in detail below.

Tirapazamine (CAS 27314-97-2; https://pubchem.ncbi.nlm.nih.gov/compound/Tirapazamine), is a compound of the following formula:

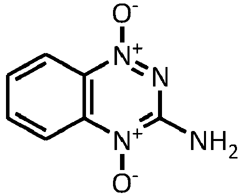

Tirapazamine has been used for the treatment of solid tumors.

The compound is commercially available, such as from Sigma Aldrich (Ref. SML0552); or may be synthesized using a suitable preparation method, such as that disclosed in GB 1 373 324 A.

Quazinone, also known as Ro 13-6438, (CAS 70018-51-8; https://pubchem.ncbi.nlm.nih.gov/compound/135418296, is a compound of the following formula:

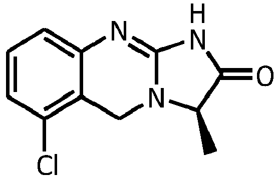

This compound has been used as a cardiotonic and vasodilator drug.

The compound is commercially available, such as from Toronto Research Chemicals (Ref.: Q509453-25mg). The compound may also be synthesized using a suitable preparation method, such as that disclosed in EP 0 000 718 A2.

The above compounds and their salts as described below may also be purchased from alternative chemical vendors, such as those found in the PubChem database maintained by the National Center for Biotechnology Information (NCBI), as can for instance be verified at the different https://pubchem.ncbi.nlm.nih.gov webpages provided herein.

In a preferred particular embodiment, the compound is Tirapazamine, or a pharmaceutically acceptable salt thereof, preferably it is Tirapazamine.

In a preferred particular embodiment, the compound is Quazinone or a pharmaceutically acceptable salt thereof, preferably Quazinone.

The term "pharmaceutically acceptable" refers to molecular entities and compositions that are physiologically tolerable and do not typically produce an allergic or similar untoward reaction, such as gastric upset, dizziness and the like, when administered to a human.

Further, the term "pharmaceutically acceptable salt" refers to any salt, which, upon administration to the recipient is capable of providing (directly or indirectly) a compound as described herein. For instance, a pharmaceutically acceptable salt of compounds provided herein may be acid addition salts or base addition salts, and they can be synthesized from the parent compound, which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, non-aqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate. Examples of the alkali addition salts include organic salts such as for example, ammonium salts, or inorganic salts such as alkali metal salts such as sodium or potassium salts.

The combinations of the present invention are combinations of Tirapazamine and Quazinone, either with each other or with other agents known to be useful in the treatment and/or prevention of a GM2 gangliosidosis. The term "agent" is used to designate either a therapeutic compound useful in the treatment and/or prevention of a GM2 gangliosidosis (such as N-acetyl-DL-leucine, RT-001, venglustat, miglustat, eliglustat, ACT-519276, ruboxistaurin, gemfibrozil, cinnamic acid or OT-1009) or a more complex therapeutic agent for example a gene therapy (such as TGTX-101, TSHA-119, AAV2/1-HEXA/HEXB, Cobnabexagene anvuparvovec, GT-0005X), a cell based treatment (such as DUOC-01) or enzyme replacement therapy (such as AX-451).

In a preferred embodiment, the combination is a combination comprising two or more agents selected from the group consisting of Tirapazamine, Quazinone, N-acetyl-DL-leucine, N-acetyl-L-leucine, RT-001, venglustat, miglustat, eliglustat, ACT-519276, ruboxistaurin, gemfibrozil, cinnamic acid, OT-1009, TGTX-101, TSHA-119, AAV2/1-HEXA/HEXB gene therapy, cobnabexagene anvuparvovec, DUOC-01, GT-0005X, AX-451, or a pharmaceutically acceptable salt thereof, wherein at least one of said agents is selected from the group consisting of Tirapazamine, Quazinone, or a pharmaceutically acceptable salt thereof.

In a preferred particular embodiment, at least one of said compounds comprised in the combination is Tirapazamine or a pharmaceutically acceptable salt thereof, as presented in any of the embodiments described above.

In a preferred particular embodiment, at least one of said compounds comprised in the combination is Quazinone or a pharmaceutically acceptable salt thereof, as presented in any of the embodiments described above.

The present invention is directed to the medical use of said compounds and combinations in the treatment and/or prevention of a GM2 gangliosidosis and/or other diseases that may benefit of an increase of beta-hexosaminidase activity, such as Alzheimer's disease and Parkinson's disease. The term "GM2 gangliosidosis", as used in the present invention, designates any disease or disorder caused by the accumulation of ganglioside GM2, in particular in neural tissue. Non-limiting examples of GM2 gangliosidoses are Tay-Sachs (TS) disease, Sandhoff disease and the AB variant of GM2 gangliosidosis.

The term ganglioside GM2 is used herein to designate (2S,3R,4E)-3-Hydroxy-2-octadecanamidooctadec-4-en-1-yl (5-acetamido-3,5-dideoxy-D-glycero-α-D-galacto-non-2-ulopyranosylonic acid)-(2→3)-[2-acetamido-2-deoxy-β-D-galactopyranosyl-(1→4)]-β-D-galactopyranosyl-(1→4)-β-D-glucopyranoside of formula:

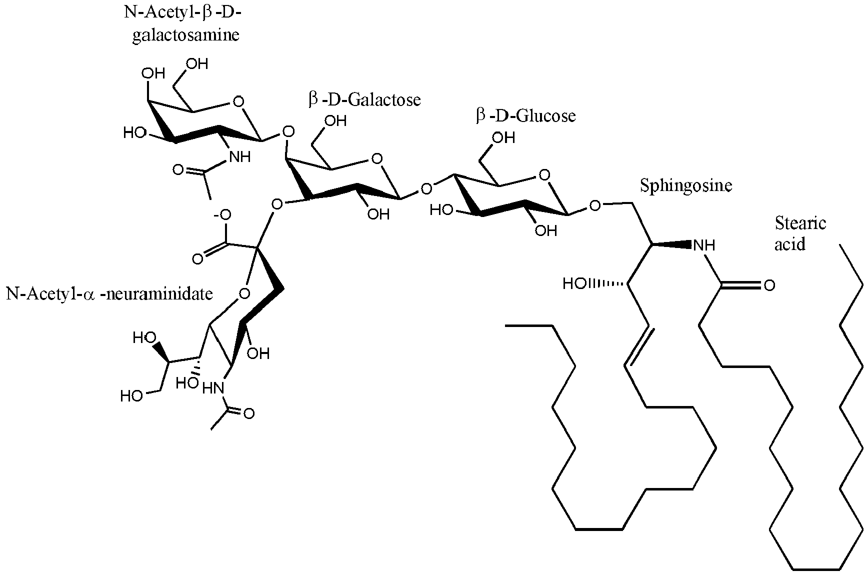

The term lyso ganglioside GM2 is used herein to designate the molecule resulting from the removal of the stearic acid in ganglioside GM2 having the following formula:

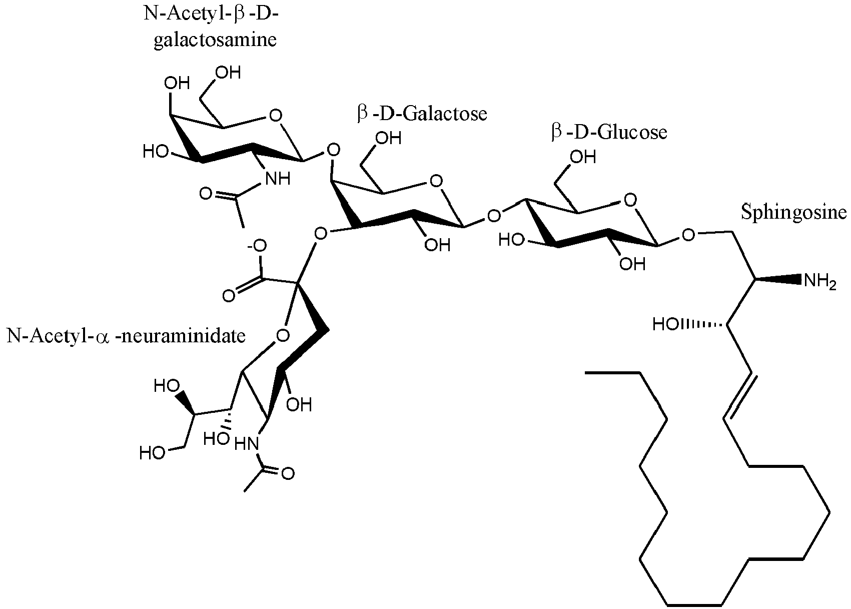

The term asialo GM2 is used herein to designate the molecule resulting from the removal of the N-acetyl-α-neuramidinate group in ganglioside GM2 having the following formula:

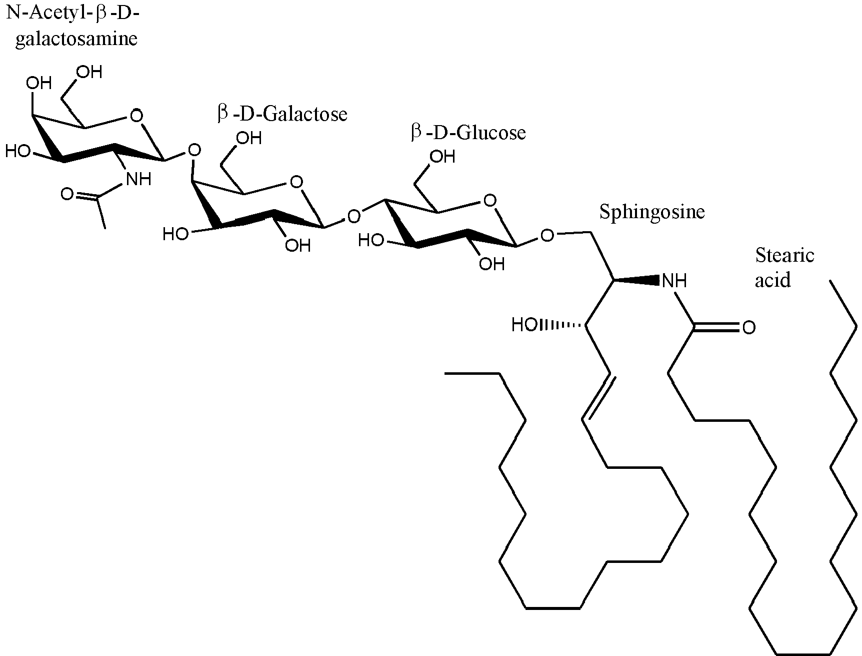

The terms "treating" and "treatment", as used herein, means reversing, alleviating, inhibiting the progress of the disease or disorder, or of one or more symptoms of said disease or disorder such as accumulation of ganglioside GM2, lyso-ganglioside GM2 and/or asialo-GM2 in cells and deterioration and/or destruction of nerve cells in the brain and/or spinal cord and/or deterioration of mental and physical abilities.

The terms "preventing" and "prevention", as used herein, means avoiding or inhibiting the onset of said disease or disorder, or of one or more of said symptoms.

The compounds for use according to the invention may be administered by any appropriate route. Non-limiting examples of administration routes are the oral, parenteral (e.g., subcutaneous, intramuscular, intravenous, etc.), topical, sublingual, inhaled, rectal, vaginal, or ophthalmic routes. In a preferred embodiment, the compounds for use according to the invention are administered by oral or parenteral route, the intravenous route being a particularly preferred parenteral route.

In particular, the compounds for use according to the invention are administered as a pharmaceutical composition, which comprises the corresponding (active) compound and one or more pharmaceutically acceptable excipients. The term "pharmaceutically acceptable excipient" refers to a vehicle, diluent, or adjuvant that is administered with the active ingredient. The identity of the pharmaceutically acceptable excipient will necessarily depend, among other factors, on the elected administration route. Said pharmaceutical compositions may be manufactured according to conventional methods known by the skilled person in the art, such as those described in European and US Pharmacopoeias.

Thus, the present invention also relates to a pharmaceutical composition comprising a compound selected from the group consisting of Tirapazamine and Quazinone, or a pharmaceutically acceptable salt thereof, or to a combination according to the present invention; and at least one pharmaceutically acceptable excipient; for use in the treatment or prevention of a GM2 gangliosidosis.

Similarly, the invention also relates to the use of a pharmaceutical composition comprising a compound selected from the group consisting of Tirapazamine and Quazinone, or a pharmaceutically acceptable salt thereof, or to a combination according to the present invention; and at least one pharmaceutically acceptable excipient; in the manufacture of a medicament for the treatment and/or prevention of a GM2 gangliosidosis.

Similarly, the invention also relates to a method of treating and/or preventing a GM2 gangliosidosis in a subject, comprising administering to said subject a therapeutically effective amount of a pharmaceutical composition comprising a compound selected from the group consisting of Tirapazamine and Quazinone, or a pharmaceutically acceptable salt thereof, or of a combination according to the present invention; and at least one pharmaceutically acceptable excipient.

In yet an additional aspect, the invention also relates to a pharmaceutical composition comprising a combination of the invention; and at least one pharmaceutically acceptable excipient.

In an embodiment, the pharmaceutical compositions are in oral delivery form. Pharmaceutical forms suitable for oral administration may be tablets, suspensions and capsules and may contain conventional excipients known in the art such as binders, for example syrup, gum arabic, gelatin, sorbitol, tragacanth or polyvinylpyrrolidone; fillers, for example lactose, sugar, cornstarch, calcium phosphate, sorbitol or glycine; lubricants for the preparation of tablets, for example magnesium stearate; disintegrants, for example starch, polyvinylpyrrolidone, sodium starch glycolate or microcrystalline cellulose; or pharmaceutically acceptable wetting agents such as sodium lauryl sulphate; sweeteners, for example sucralose or aspartame and/or taste-masking agents such as flavors (for example orange, strawberry or mint), bitterness blocking agents (such as adenosine monophosphate, lipoproteins, or phospholipids) and effervescent agents (for example sodium bicarbonate or citric acid). Solid oral compositions can be prepared by conventional methods such as blending, filling or compressing.

In an embodiment, the pharmaceutical compositions are in parenteral, preferably intravenous, delivery form.

Pharmaceutical forms suitable for parenteral administration may be sterile solutions or suspensions based on aqueous vehicles such as water or sodium chloride, Ringer or dextrose solutions; water-miscible vehicles including, but not limited to, ethyl alcohol, polyethylene glycol and polypropylene glycol; or non-aqueous vehicles including, but not limited to, corn oil, cottonseed oil, peanut oil, sesame oil, ethyl oleate, isopropyl myristate and benzyl benzoate. Suitable excipients such as fillers, buffering agents or surfactants can be used.

The compounds for use according to the invention are preferably administered in or comprised in the pharmaceutical compositions or combinations in a "therapeutically effective amount", i.e. a nontoxic but sufficient amount of the corresponding compound to provide the desired effect, namely the treatment and/or prevention of a GM2 gangliosidosis. The amount that is "effective" will vary from subject to subject, depending on the age and general condition of the individual, the particular compound administered, and the like. Thus, it is not always possible to specify an exact "therapeutically effective amount". However, an appropriate amount in any individual case may be determined by one of ordinary skill in the art using routine experimentation.

The term "subject" preferably refers to a mammal, such as a human.

The compounds for use according to the invention will typically be administered once or more times a day, for example 1, 2, 3 or 4 times daily, with typical total daily doses depending on the particular compound and severity of the disease, and may be easily determined by the skilled practitioner.

Typical total oral daily doses of tirapazamine, or a pharmaceutically acceptable salt thereof, are in the range of from 2 to 1000 mg/day (expressed as tirapazamine free base), preferably from 10 to 150 mg/day. Typical total intravenous doses of tirapazamine, or a pharmaceutically acceptable salt thereof, are in the range of from 10 to 800 mg every 21 days or 3 times per week (expressed as tirapazamine free base), preferably from 100 to 700 mg every 21 days or 3 times per week.

Typical total oral daily doses of quazinone, or a pharmaceutically acceptable salt thereof, are in the range of from 1 to 600 mg/day (expressed as quazinone free base), preferably from 10 to 60 mg/day. Typical total intravenous daily doses of quazinone, or a pharmaceutically acceptable salt thereof, are in the range of from 0.1 to 200 mg (expressed as quazinone free base), preferably from 1 to 20 mg.

For pediatric patients, doses will be adapted to the corresponding mg/kg dose as determined by the skilled practitioner.

The compounds for use according to the invention may be administered as the sole active ingredient; or in combination with other active ingredients as is described elsewhere herein. In a particular embodiment, the compounds are used as the sole active ingredient. In another particular embodiment, the compounds are used in combination with other active ingredients as is described elsewhere herein.

The term "combination" refers to a product comprising one or more of the defined agents, either in a single composition or in several compositions (or units), in which case the corresponding agents are distributed among the several compositions.

When the combination is in the form of a single composition, the agents present in the combination are always administered simultaneously.

When the combination is in the form of several compositions (or units), each of them having at least one of the agents of the combination, the compositions or (units) may be administered simultaneously, sequentially or separately.

Simultaneous administration means that the agents or compositions (or units) are administered at the same time.

Sequential administration means that the agents or compositions (or units) are administered at different time points in a chronologically staggered manner.

Separate administration means that the agents or compositions (or units) are administered at different time points independently of each other.

The combinations for use according to the invention will typically be administered once or more times a day, for example 1, 2, 3 or 4 times daily, with typical total daily doses depending on the particular agent and severity of the disease, and may be easily determined by the skilled practitioner.

Tirapazamine and quazinone and/or pharmaceutically acceptable salts have been described in detail above.

N-acetyl-DL-leucine, also known as IB 1001 or acetylleucine (CAS 99-15-0; https://pubchem.ncbi.nlm.nih.gov/compound/1995), has the chemical structure depicted below.

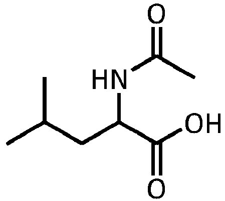

This compound acts as a neuroprotectant and it is in development for the treatment of GM2 gangliosidoses. The compound is commercially available, such as from MedChemExpress (Ref. HY-B1442).

N-Acetyl-L-leucine (CAS 1188-21-2; https://pubchem.ncbi.nlm.nih.gov/compound/70912), has the chemical structure depicted below.

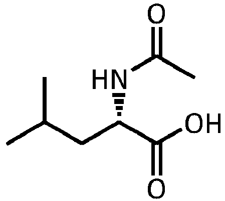

This compound acts as a neuroprotectant and it is in development for the treatment of GM2 gangliosidoses. The compound is commercially available, such as from MedChemExpress (Ref. HY-59291).

RT-001 (CAS 1404475-07-5; https://pubchem.ncbi.nlm.nih.gov/compound/124037379), has the chemical structure depicted below.

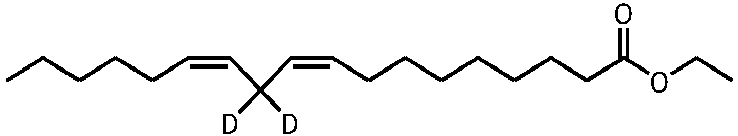

This compound is a lipid peroxidation inhibitor that is in development for the treatment of GM2 gangliosidoses. The compound is commercially available, such as from CombiPhos (Ref. MS58); or may be synthesized using a suitable preparation method, such as that disclosed in WO 2017/091279 A1.

Venglustat, also known as ibiglustat (CAS 1401090-53-6; https://pubchem.ncbi.nlm.nih.gov/compound/60199242), has the chemical structure depicted below.

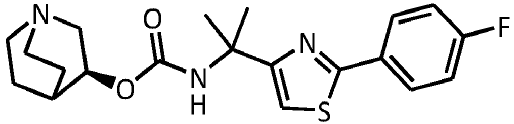

This compound is a glucosylceramide synthase (GCS) inhibitor that is in development as a malate salt (venglustat malate, CAS 1629063-78-0) for the treatment of GM2 gangliosidoses and other lysosomal storage disorders.

The compound is commercially available, such as from MedChemExpress (ref. HY-16743); or may be synthesized using a suitable preparation method, such as that disclosed in WO 2020/163337 A1.

Miglustat, commercialized under the brand name Zavesca (CAS 72599-27-0; https://pubchem.ncbi.nlm.nih.gov/compound/51634), has the chemical structure depicted below.

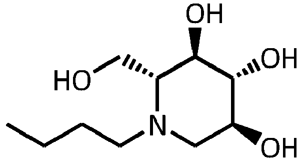

This compound is a glucosylceramide synthase (GCS) inhibitor that is commercialized for the treatment of Gaucher disease and Niemann Pick C. Miglustat is in development for the treatment of lysosomal storage disorders such as GM2 gangliosidoses.

The compound is commercially available, such as from MedChemExpress (ref. HY-17020); or may be synthesized using a suitable preparation method, such as that disclosed in WO 91/17145 A1.

Eliglustat, commercialized under the brand name Cerdelga (CAS 491833-29-5; https://pubchem.ncbi.nlm.nih.gov/compound/23652731), has the chemical structure depicted below.

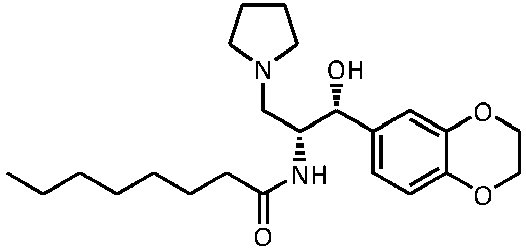

This compound is a glucosylceramide synthase (GCS) inhibitor that is commercialized for the treatment of Gaucher disease.

The compound is commercially available, such as from MedChemExpress (ref. HY-14885); or may be synthesized using a suitable preparation method, such as that disclosed in WO 2003/008399 A1.

ACT-519276, also known as sinbaglustat (OGT-2378) (CAS 441061-33-2; https://pubchem.ncbi.nlm.nih.gov/compound/9859470), has the chemical structure depicted below.

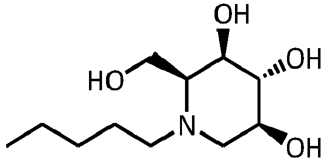

This compound is a dual glucosylceramidase beta 2 (GBA2) and glucosylceramide synthase (GCS) inhibitor that is in development for the treatment of GM2 gangliosidoses. The compound may be synthesized using a suitable preparation method, such as that disclosed in WO 02/055498 A1.

TGTX-101, also known as TSHA-101, is a gene therapy agent that is in development by Taysha Gene Therapies Inc for the treatment of GM2 gangliosidoses. The product is a bicistronic, adeno-associated virus (AAV) serotype 9 vector carrying both HEXA and HEXB genes under the control of a CAG promotor. The agent may be synthesized using a suitable preparation method, such as that disclosed in Woodley et al Molecular Therapy--Methods & Clinical Development. Volume: 12. Pages: 47-57. https://doi.org/10.1016/j.omtm.2018.10.011.

TSHA-119 is an adeno-associated virus serotype 9 (AAV9) capsid gene therapy, for the potential intrathecal treatment of GM2 gangliosidosis AB variant. This treatment is in development by Taysha Gene Therapies Inc. The agent may be synthesized using a suitable preparation method, such as that disclosed in Karumuthil-Melethil, et al. Human Gene TherapyVol. 27, No. 7. https://doi.org/10.1089/hum.2016.013.

AAV2/1-HEXA/HEXB gene therapy is a recombinant adeno-associated virus (AAV) serotype 2/1 vector encoding human beta-hexosaminidase alpha and beta subunits. The product is in development by University of Cambridge for the treatment of GM2 gangliosidoses. The agent may be synthesized using a suitable preparation method, such as that disclosed in Cachón-González et al PNAS July 5, 2006 103 (27) 10373-10378. https://doi.org/10.1073/pnas.0603765103.

Cobnabexagene anvuparvovec, also known as AXO-AAV-GM2, is a recombinant adeno-associated virus vector (AAV2/rh8) expressing human beta-hexosaminidase alpha (HEXA) and beta (HEXB). This gene therapy agent is in development by Sio Gene Therapies Inc for the treatment of GM2 gangliosidoses. The agent may be synthesized using a suitable preparation method, such as that disclosed in Golebiowski et al Human Gene Therapy Vol. 28, No. 6. https://doi.org/10.1089/hum.2016.109.

DUOC-01 is a cell based treatment prepared from banked cord blood that is in development by Duke University for the treatment of GM2 gangliosidosis and demyelinating diseases. The agent may be synthesized using a suitable preparation method, such as that disclosed in US 2021/0275583A1.

Ruboxistaurin, also known as REC-3599 (CAS 169939-94-0; https://pubchem.ncbi.nlm.nih.gov/compound/153999), has the chemical structure depicted below.

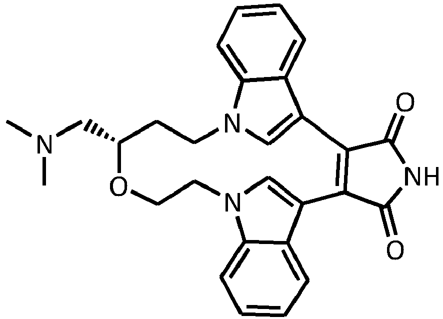

This compound is an inhibitor of glycogen synthase kinase 3 beta (GSK3b) and protein kinase C (PKC) that is in development for the treatment of GM2 gangliosidoses. The compound is commercially available, such as from MedChemExpress (ref. HY-10195); or may be synthesized using a suitable preparation method, such as that disclosed in EP 0 657 458 A1.

Gemfibrozil, also known as PLX-200 (CAS 25812-30-0; https://pubchem.ncbi.nlm.nih.gov/compound/3463), has the chemical structure depicted below.

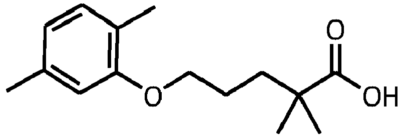

This compound is a lipid lowering drug that acts as a retinoid X receptor alpha (RXRA) and peroxisome proliferator-activated receptor alpha (PPARA) agonist increasing lysosome biogenesis. The compound is in development for the treatment of GM2 gangliosidoses.

The compound is commercially available, such as from MedChemExpress (ref. HY-B0258); or may be synthesized using a suitable preparation method, such as that disclosed in WO 94/27948 A1.

Cinnamic acid, also known as PLX-300 (CAS 621-82-9; https://pubchem.ncbi.nlm.nih.gov/compound/444539), has the chemical structure depicted below.

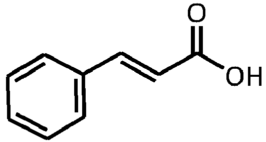

This compound is a peroxisome proliferator-activated receptor alpha (PPARA) agonist that reduces neuroinflammation and neurotoxicity. The compound is in development for the treatment of GM2 gangliosidoses. The compound is commercially available, such as from MedChemExpress (ref. HY-N0610A); or may be synthesized using a suitable preparation method, such as that disclosed in US 4,571,432.

GT-0005X is a gene therapy using recombinant adeno-associated virus (AAV). The product is in development by Gene Therapy Research Institution Co Ltd for the treatment of GM2 gangliosidoses. The agent may be synthesized using a suitable preparation method, such as that disclosed in WO 2019/146745 A1.

AX-451 is an enzyme replacement therapy for CNS delivery in development by Allievex Corp for the treatment of GM2 gangliosidoses.

OT-1009, also known as OT-1001 (CAS 958868-91-2), has the chemical structure depicted below.

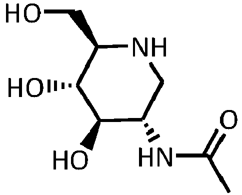

This compound is a pharmacological chaperone that acts by targeting and stabilizing beta-hexosaminidase. The compound is in development for the treatment of GM2 gangliosidoses by Amicus Therapeutics and Orphi Therapeutics Inc. The compound is commercially available, such as from Aurora Building Blocks 4 (ref. 132.104.392).

The following examples represent specific embodiments of the present invention. They do not intend to limit in any way the scope of the invention defined in the present description.

### Examples

### Materials and methods

### 1. Cell lines

Human fibroblast cell line GM00502 was obtained from a 1-year-old male Tay-Sachs patient that was compound heterozygote with a 4-bp insertion at nucleotide 1278 in exon 11 of the HEXA gene (Gene ID: 3073, updated on 26-Nov-2021 - https://www.ncbi.nlm.nih.gov/gene/3073) [1278insTATC] causing a premature termination signal and deficiency of mRNA in one allele, and a G>C splice mutation in intron 12 of the second allele (IVS12+1G>C). Cells were grown as monolayers in T25 and T75 tissue culture coated tanks in a Thermo humidified CO2 incubator maintained at 37°C in Dulbecco's Modified Eagle's Medium (DMEM) supplemented with 10% Foetal Bovine Serum (FBS) and 1% L-glutamine.

### 2. Test compounds

Reagents were purchased at Sigma-Aldrich. Tirapazamine and quazinone were purchased at Sigma-Aldrich and Santa Cruz Biotechnology, respectively. Compounds were solubilised in dimethylsulfoxide (DMSO) to 100mM. A working DMSO stock at a concentration of 3mg/ml was prepared for drug treatment. The stock was diluted appropriately according to the desired treatment concentrations (3µg/ml). DMSO control was used in all assays (vehicle).

### 3. Cell treatments

Once cells were >90% confluent in T75 flasks they were split into T25s for enzyme assay. Cells were then treated with either vehicle (DMSO), tirapazamine or quazinone for 10 days. Fresh, complete media with vehicle or compounds was changed every other day to maintain drug efficacy in cell lines. Following treatment period cells were detached from T25 flasks by 0.25% trypsin/EDTA. Trypsin was inactivated by adding DMEM, and cells pelleted by centrifugation at 102 g for 5 minutes. Medium was removed, and cells were resuspended in DPBS. The centrifugation and DPBS wash steps were repeated twice, and cells were stored as dry pellets at -80°C.

4. Bicinchoninic acid assay: Bicinchoninic acid (BCA) assays were used to obtain the total concentration of proteins present in the cellular samples. BCA Protein Assay (Sigma Aldrich) was conducted according to the manufacturers' instructions. Briefly, cell pellets were resuspended in MilliQ water (Barnstead purifier,ThermoFisher), and cells fractured by three freeze-thaw cycles. 50µl of homogenate was then taken and centrifuged at ~13,000 g for 30 minutes to pellet insoluble material. The supernatant was collected and diluted 1:5, 1:10 and 1:20 for determination of protein concentration, using 0-1mg/ml BSA as the standard curve. Samples were plated onto clear, flat bottomed 96 well microplates (Greiner Bio-one, Austria) and incubated with BCA reagent (0.02% Cu2SO4 in bicinchoninic acid solution) for 30 minutes at 37°C to allow the colour to develop. Absorbance at 570nm was measured using a TECAN Infinite F50 absorbance microplate reader (Tecan Group Ltd. Switzerland). To minimize the effect of thawing on enzyme activity, multiple single- use aliquots were prepared following determination of protein concentration, and these were stored at -80°C.

5. Beta-hexosaminidase fluorometric assay: To determine beta-hexosaminidase activity in cell homogenates, 10µl cell homogenate (containing 1µg protein) and 10µl artificial fluorogenic substrate 4MU-N-acetyl-α-D-glucosamine (1 mM final concentration), were added to clear, flat bottomed 96-well microplates (Greiner Bio-one), and incubated for 4 hours at 37°C. Samples and substrate were prepared in working buffer (citrate buffer, pH 4.6 + 0.01% Triton X-100). The standard was prepared in the working buffer at concentrations ranging between 0 - 200µM. The reaction was stopped by adding 180µl of Na₂CO₃, pH 10, and the released fluorophore measured using a SpectraMAX Gemini EM fluorescence plate reader (Molecular Devices) at an excitation of 360nm and emission of 460 nm. The enzyme activity was calculated using µmol/hour. Heatinactivated control samples were used to measure background fluorescence in the enzyme activity assays and subtracted from the original reading. During this process, samples were heated to 95°C for 1 hour to achieve protein denaturation. Experiments were carried out with 3 independent samples, statistical analysis (Anova) was performed with Graph Pad Prism 8.

### Results

The data obtained analyzing the effects of tirapazamine and quazinone in Tay-Sachs GM0502 cells showed that the compounds induce a significant increase in b-Hex activity in Tay-Sachs patient fibroblasts after 10 days' treatment (Figure 1). These results indicate that these compounds have potential for the prevention and/or treatment of diseases associated with b-Hex deficiency.

## Claims

1. Compound selected from the group consisting of Tirapazamine and Quazinone, or a pharmaceutically acceptable salt thereof, for use in the treatment or prevention of a GM2 gangliosidosis.

2. Compound for use according to claim 1, wherein the compound is Tirapazamine or a pharmaceutically acceptable salt thereof.

3. Compound for use according to claim 1, wherein the compound is Quazinone or a pharmaceutically acceptable salt thereof.

4. Combination comprising two or more agents selected from the group consisting of Tirapazamine, Quazinone, N-acetyl-DL-leucine, N-acetyl-L-leucine, RT-001, venglustat, miglustat, eliglustat, ACT-519276, ruboxistaurin, gemfibrozil, cinnamic acid, OT-1009, TGTX-101, TSHA-119, AAV2/1-HEXA/HEXB gene therapy, cobnabexagene anvuparvovec, DUOC-01, GT-0005X, AX-451 or a pharmaceutically acceptable salt thereof, wherein at least one of said agents is selected from the group consisting of Tirapazamine and Quazinone, or a pharmaceutically acceptable salt thereof.

5. Combination as defined in claim 4, for use in the treatment or prevention of a GM2 gangliosidosis.

6. Pharmaceutical composition comprising:
- a compound selected from the group consisting of Tirapazamine and Quazinone, or a pharmaceutically acceptable salt thereof; or a combination as defined in claim 4; and
- at least one pharmaceutically acceptable excipient,
for use in the treatment or prevention of a GM2 gangliosidosis.

7. A pharmaceutical composition for use according to claim 6, wherein the pharmaceutical composition is administered by oral or intravenous route.

8. Compound for use according to any one of claims 1 to 3, or combination for use according to claim 5, or pharmaceutical composition for use according to any one of claims 6 to 7, wherein the GM2 gangliosidosis is selected from Tay-Sachs (TS) disease, Sandhoff disease and the AB variant of GM2 gangliosidosis.
